# EUROPEAN PATENT APPLICATION

(11) **EP 2 316 422 A1**
(43) Date of publication of application: **04.05.2011**
(21) Application number: 10177744.9
(22) Date of filing: 10.11.2008
(51) Int. Cl.: A61K 9/00, A61K 31/41

(54) **Liquid compositions comprising valsartan**

(30) Priority: 12.11.2007 US 987178 P
(62) Divisional of application: 08850984.9
(71) Applicant: Novartis AG, 4056 Basel (CH)
(72) Inventor: Harasymiw, Gregory, Morristown, NJ 07960 (US); Talamonti, Wayne, Kunkletown, PA 18058 (US); Wagner, Robert Frank, Hillsborough, NJ 08844 (US); Zielinski, Joseph Lawrence, Florham Park, NJ 07932 (US)
(74) Representative: Barbier, Denis Robert

(57) **Abstract**

*Provided are compositions comprising aqueous solutions of valsartan suitable for oral administration, preferably in pediatric and geriatric populations. Methods for making such compositions and methods for their stabilization are provided.*

## Description

This invention relates to pharmaceutical compositions for the treatment of angiotensin II mediated disorders and conditions comprising valsartan or a pharmaceutically acceptable salt thereof suitable for oral administration, and methods of treatment of angiotensin II mediated disorders and conditions by the oral administration of such pharmaceutical compositions of valsartan.

All patents, patent applications, and other publications referred to herein are hereby expressly incorporated by reference in their entirety. In case of a conflict between the present specification and material incorporated by reference, the present specification is controlling.

The present invention is directed to a composition for the treatment of angiotensin II mediated disorders and conditions, the composition comprising a solution of valsartan. Valsartan or ((S)-*N-*valeryl-*N-*{[2'-(1*H-*tetrazole-5-yl)-biphenyl-4-yl]-methyl}-valine) suitable for use in the present invention can be purchased from commercial sources or can be prepared according to known methods. For example, the preparation of Valsartan is described in U.S. Patent No. 5,399,578, the entire disclosure of which is incorporated by reference herein. Valsartan may be used for purposes of this invention in its free form as well as in any suitable salt form.

Also included within the scope of the present invention are the salts, esters, amides, prodrugs, active metabolites, analogs, and the like of Valsartan, particularly the calcium salt of Valsartan. A detailed description of the calcium salt and process of making are disclosed in published U.S. Patent Application No. 2003/0207930, the contents of which are fully incorporated by reference herein in their entirety.

The present invention is also directed to methods for treating an angiotensin II mediated disorder or condition comprising administering an effective amount of the compositions of the invention, i.e., a liquid oral dosage formulation comprising valsartan.

As discussed in U.S. Patent 5,399,578, a genus of compounds, including valsartan, may be employed to treat hypertension, congestive heart failure, angina, myocardial infarction, arteriosclerosis, diabetic nephropathy, diabetic cardiac myopathy, renal insufficiency, peripheral vascular disease, stroke, left ventricular hypertrophy, cognitive dysfunction, headache, or chronic heart failure by administering a therapeutically effective amount of the pharmaceutical compositions of the present invention to a subject in need of such treatment.

Some individuals, especially children and geriatrics, have difficulty swallowing solid oral dosage formulations. Moreover, flexibility in mg/kg dosing is required for the pediatric and geriatric population. Thus, it is desirable to provide stable long-lasting liquid oral dosage formulations comprising valsartan for the treatment of the aforementioned conditions in individuals who have difficulty swallowing solid oral dosage formulations.

It has now surprisingly been found that a shelf-stable liquid oral dosage formulation comprising valsartan can be prepared. The valsartan drug substance is relatively water insoluble and also degrades in water, and so the ability to produce a shelf-stable formulation was unexpected. The liquid oral dosage formulations comprising valsartan are preferably solutions of valsartan. Valsartan concentration is between about 1 mg/ml to about 5 mg/ml, preferably about 3 mg/ml. The formulations of the invention can also contain a wetting agent, e.g., polysorbate 80, poloxamers, including poloxamer 188, polyethoxylated castor oil and polyethoxylated hydrogenated castor oil, and polyoxyl 40 stearate. Poloxamer 188 has the structure HO(CH₂CH₂O)ₐ(CH(CH₃)CH₂OH)_{b}(CH₂CH₂O)_{c}H, where a is 75, b is 30, and c is 75, with an average molecular weight of about 8350. The wetting agent is present in amounts typically between about 0.1 % and about 5%, or between about 0.2% and about 1%, or between about 0.5%

The pH of the formulation can range between about 4.5 and .7.0, preferably between about 5.5 and about 6.5 even more preferably between about 5.5 and about 6.0 or between about 5.7 and about 6.2, most preferably about 5.9. Suitable buffers include, e.g.., alkaline metal citrate buffers, such as alkaline metal citrate salts with citric acid, alkaline metal acetate buffers, such as sodium acetate salts with acetic acid, and alkaline metal succinate buffers, such as sodium succinate salts with succinic acid, and mixtures thereof. Preferred buffers include citric acid and sodium citrate.

The formulations typically contain an antifoaming agent, e.g., simethicone, typically added as an emulsion, e.g., a 30% emulsion. Such a 30% emulsion can be added at a concentration of about 0.1% to about 0.25% in the final formulation. Sweeteners such as mannitol, sucralose, saccharin, sodium saccharin, aspartame, sucralose, acesulfame potassium, glucose, fructose, lactitol, maltitol, maltose, sorbitol, sucrose, and xylitol can be used. Flavoring agents can also be added to improve compliance.

Suitable preservatives for oral solutions are known to those of skill in the art and include, e.g., benzoic acid, sorbic acid (and salts thereof), parabens (butyl, ethyl, methyl, propyl), sodium benzoate, and sodium propionate. A preservative such as those set forth above, or a mixture thereof, can be present in amounts between about 0.01% and about 0.5%; or between about 0.02% and 0.25%; or between about 0.1% and about 0.2%. In one embodiment, the formulation comprises about 0.02% propyl paraben and about 0.18% methyl paraben. Other embodiments include formulations comprising 0.03% propyl paraben and 0.12% methyl paraben, 0.148% methylparaben and 0.016% propylparaben and formulations comprising 0.16% methyl paraben and 0.2% potassium sorbate.

The solutions of the invention can be made in conventional liquid formulation equipment. In one embodiment, the solution of the invention is produced by a process comprising admixing water, drug substance, followed by the addition and admixture of buffer components, sweeteners, and flavouring agents. Alternatively, preservatives, sweetener, flavour, and the buffer components are dissolved in water. Separately, methylparaben and valsartan drug substance may be dissolved in propylene glycol, with heat, to form a solution. This propylene glycol solution is then admixed with the aqueous portion and a final solution is prepared. The buffer components can be adjusted to produce the desired solution pH. A pH of between about 4.5 and 7.0 provides a solution with the most stable drug substance.

The following examples are illustrative, but do not serve to limit the scope of the invention described herein. The examples are meant only to suggest a method of practicing the present invention.

### Examples

### Example 1: Formulation

**Table 1**

| **Table 1** | **Composition of Diovan oral solution** | | | | | |
|---|---|---|---|---|---|---|
| **Ingredients** | **mg/ml** | **Quantity per 1,000 liters** | **Function** | **NOPAS number** | **Huningue code no.** | **Reference to standard** |
| VAL489 DS | 1.0 | 1.0 kg | Drug substance | 133730 | | Novartis monograph |
| Potassium sorbate | 2.0 | 2.0 | Preservative | | | Ph. Eur.; NF |
| Poloxamer 188. NF | 5.00 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Peach flavor | 1.0 | 1.0 | Flavoring agent | | | Novartis monograph |
| Sucrose | 300.0 | 300.0 | Sweetener | | | Ph. Eur.; USP |
| citric acid, anhydrous, USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified, USP | Qs 1.0ml | QS, 1116 kg | Vehicle | 115761 | 20442 | Ph. Eur.: USP |

Poloxamer 188, preservatives, sweetener, flavour, and the buffer components are dissolved in water, followed by the addition of the valsartan drug substance, with heat up to 90°C, and a solution formed. The following formulations can be prepared as indicated above, using the following ingredients:

**Table 2 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 830720 | 830720 | Novartis monograph |
| Potassium sorbate | 2.0 | 2.0 | Preservative | 970086 | 1320047 | Ph. Eur.; NF |
| Poloxamer 188, NF | 5.00 | 5.0 | Wetting agent | 118209 | 28018 | Ph. Eur.; NF |
| Methylparaben, NF | 1.62 | 1.62 | Preservative | 100203 | 28014 | Ph. Eur.; NF |
| Blueberry flavor | 1.0 | 1.0 | Flavoring agent | 970254 | 1320076 | Novartis monograph |
| Sucrose | 300.0 | 300.0 | Sweetener | 970250 | 25143 | Ph. Eur.; USP |
| citric acid, anhydrous, USP | 0.67 | 1.02 | Buffer | 115442 | 28017 | Ph. Eur.; USP |
| sodium citrate, dihydrate, USP | 15.84 | 13.12 | Buffer | 115610 | 20003 | Ph. Eur.; USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 5.9 | | | | | | |

**Table 3 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 | | Novartis monograph |
| Propyl paraben | 0.18 | 0.18 | Preservative | | | Ph. Eur.; NF |
| Propylene glycol | 25.0 | 25.0 | Wetting agent | 108695 | 28018 | Ph. Eur.; NF |
| Methytparaben, NF | 1.62 | 1.62 | Preservative | 108353 | 28014 | Ph. Eur.; NF |
| Grape flavor | 3.0 | 3.0 | Flavoring agent | | | Novartis monograph |
| Sucrose | 300.0 | 300.0 | Sweetener | | | Ph. Eur.; USP |
| citric acid, anhydrous, USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified, USP | Qs 1.0ml | QS, 1116 kg | Vehicle | 115761 | 20442 | Ph. Eur.; USP |

**Table 4 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 | | Novartis monograph |
| Propyl paraben | 0.18 | 0.18 | Preservative | | | Ph. Eur.; NF |
| Polyethylene glycol 400 | 25.0 | 25.0 | Wetting agent | 108695 | 28018 | Ph. Eur.; NF |
| Methylparaben, NF | 1.62 | 1.62 | Preservative | 108353 | 28014 | Ph. Eur.; NF |
| grape flavor | 3.0 | 3.0 | Flavoring agent | | | Novartis monograph |
| Sucrose | 300.0 | 300.0 | Sweetener | | | Ph. Eur.; USP |
| citric acid, anhydrous. USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified, USP | Qs 1.0ml | QS, 1116kg | Vehicle | 115761 | 20442 | Ph. Eur.; USP |

| | | | | | | |
|---|---|---|---|---|---|---|
| pH 6 | | | | | | |

**Table 5 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 5.0 | 5.0 kg | Drug substance | 133730 | | Novartis monograph |
| Potassium sorbate | 2.0 | 2.0 | Preservative | | | Ph. Eur.; NF |
| Poloxamer 188, NF | 5.00 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Peach flavor | 1.0 | 1.0 | Flavoring agent | | | Novartis monograph |
| Sucrose | 300.0 | 300.0 | Sweetener | | | Ph. Eur.: USP |
| citric acid, anhydrous, USP | 1.02 | 1.02 | Buffer | 115442 | 28017 | Ph. Eur.; USP |
| sodium citrate, dihydrate, USP | 13.12 | 13.12 | Buffer | 115610 20003 | | Ph. Eur.; USP |
| Water purified. USP | Qs 1.0ml | QS, 1116 kg | Vehicle | 115761 | 20442 | Ph. Eur.; USP |

| | | | | | | |
|---|---|---|---|---|---|---|
| pH 6 | | | | | | |

**Table 6 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 | | Novartis monograph |
| Potassium sorbate | 2.0 | 2.0 | Preservative | | | Ph. Eur.: NF |
| Poloxamer 188, NF | 5.00 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Peach flavor | 1.0 | 1.0 | Flavoring agent | | | Novartis monograph |
| Sucrose | 300.0 | 300.0 | Sweetener | | | Ph. Eur.; USP |
| citric acid, anhydrous, USP | 1.83 | 1.83 | Buffer | 115442 | 28017 | Ph. Eur.; USP |
| sodium citrate, dihydrate, USP | 11.89 | 11.89 | Buffer | 115610 | 20003 | Ph. Eur.; USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 5.5 | | | | | | |

**Table 7 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 3.0 | 3.0 kg | Drug substance | 133730 | | Novartis monograph |
| Potassium sorbate | 2.0 | 2.0 | Preservative | | | Ph. Eur.; NF |
| Poloxamer 188, NF | 5.00 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Peach flavor | 1.0 | 1.0 | Flavoring agent | | | Novartis monograph |
| Sucrose | 300.0 | 300.0 | Sweetener | | | Ph. Eur.; USP |
| citric acid, anhydrous, USP | 3.2 | 3.2 | Buffer | 115442 | 28017 | Ph. Eur.; USP |
| sodium citrate, dihydrate, USP | 11.17 | 11.17 | Buffer | 115610 | 20003 | Ph. Eur.: USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 5.0 | | | | | | |

**Table 8 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 3.0 | 3.0 kg | Drug substance | 133730 | | Novartis monograph |
| Potassium Sorbate | 2.0 | 2.0 | Preservative | | | Ph. Eur.; NF |
| Propylene glycol | 25.0 | 25.0 | Wetting agent | 108695 | 28018 | Ph. Eur.; NF |
| Methylparaben, NF | 1.62 | 1.62 | Preservative | 108353 | 28014 | Ph. Eur.; NF |
| blueberry flavor | 1.0 | 1.0 | Flavoring agent | | | Novartis monograph |
| Sucrose | 300.0 | 300.0 | Sweetener | | | Ph. Eur.; USP |
| citric acid. anhydrous, USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified. USP | Qs 1.0ml | QS, 1116 kg | Vehicle | 115761 | 20442 | Ph. Eur.; USP |

| | | | | | | |
|---|---|---|---|---|---|---|
| _{P}H5.7 | | | | | | |

**Table 9 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 | | Novartis monograph |
| Potassium sorbate | 2.0 | 2.0 | Preservative | | | Ph. Eur.; NF |
| Poloxamer 188 | 5.0 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Blueberry flavor | 1.0 | 1.0 | Flavoring agent | | | Novartis monograph |
| Sucrose | 300.0 | 300.0 | Sweetener | | | Ph. Eur.; USP |
| citric acid, anhydrous, USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified. USP | Qs 1.0ml | QS, 1116 kg | Vehicle | 115761 | 20442 | Ph. Eur.; USP |

| | | | | | | |
|---|---|---|---|---|---|---|
| _{P}H 5.7 | | | | | | |

**Table 10 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0kg | Drug substance | 133730 | | Novartis monograph |
| Potassium sorbate | 2.0 | 2.0 | Preservative | | | Ph. Eur.: NF |
| Poloxamer 188 | 5.0 | 5.0 | Wetting agent | 108695 | 28018 | Ph. Eur.; NF |
| Methylparaben, Nf | 1.62 | 1.62 | Preservative | 108353 | 28014 | Ph. Eur.; NF |
| Blueberry flavor | 1.0 | 1.0 | Flavoring agent | | | Novartis monograph |
| Sodium saccharine | 0.5 | 0.5 | Sweetener | | | Ph. Eur.; USP |
| citric acid, anhydrous, USP | 3.2 | 3.2 | Buffer | 115442 | 28017 | Ph.Eur.; USP |
| sodium citrate, dihydrate, USP | 11.17 | 11.17 | Buffer | 115610 | 20003 | Ph. Eur.; USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 4.7 | | | | | | |

**Table 11 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 3.0 | 3.0 kg | Drug substance | 133730 | | Novartis monograph |
| Potassium sorbate | 2.0 | 2.0 | Preservative | | | Ph. Eur.; NF |
| Poloxamer 188 | 5.0 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Blueberry flavor | 1.0 | 1.0 | Flavoring agent | | | Novartis monograph |
| Sucrose | 300.0 | 300.0 | Sweetener | | | Ph. Eur.; USP |
| citric acid, anhydrous, USP | 1.02 | 1.02 | Buffer | 115442 | 28017 | Ph. Eur.; USP |
| sodium citrate. dihydrate, USP | 13.12 | 13.12 | Buffer | 115610 | 20003 | Ph. Eur.; USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 5.7 | | | | | | |

**Table 12 Composition of Diovan oral solution**

| **Ingredients** | **mg/ml** | **Quantity per 1,000 liters** | **Function** | **NOPAS number** | **Huningue code no.** | **Reference to standard** |
|---|---|---|---|---|---|---|
| VAL489 DS | 1.0 | 1.0 kg | Drug substance | 133730 | | Novartis monograph |
| Potassium sorbate | 2.0 | 2.0 | Preservative | | | Ph. Eur.; NF |
| Poloxamer 188 | 5.0 | 5.0 | Wetting agent | 108695 | 28018 | Ph. Eur.; NF |
| Methylparaben, NF | 1.62 | 1.62 | Preservative | 108353 | 28014 | Ph. Eur.; NF |
| Blueberry flavor | 1.0 | 1.0 | Flavoring agent | | | Novartis monograph |
| Sucrose | 300.0 | 300.0 | Sweetener | | | Ph. Eur.; USP |
| citric acid, anhydrous. USP | 1.02 | 1.02 | Buffer | 115442 | 28017 | Ph. Eur.; USP |
| sodium citrate, dihydrate, USP | 13.12 | 13.12 | Buffer | 115610 | 20003 | Ph. Eur.; USP |
| Water purified, USP | Qs 1.0ml | QS, 1116 kg | Vehicle | 115761 | 20442 | Ph. Eur.; USP |

| | | | | | | |
|---|---|---|---|---|---|---|
| pH 6.5 | | | | | | |

It is understood that while the present invention has been described in conjunction with the detailed description thereof that the foregoing description is intended to illustrate and not limit the scope of the invention, which is defined by the scope of the following claims. Other aspects, advantages and modifications are within the scope of the claims.

## Claims

1. A liquid oral dosage formulation comprising water, and valsartan, wherein the pH of said formulation is between about 4.5 and about 7.0.

2. The liquid oral dosage formulation of claim 1, further comprising a wetting agent.

3. The liquid oral dosage formulation of claim 2, wherein said wetting agent is a member selected from the group consisting of polysorbate 80, poloxamers, polyethoxylated castor oil, polyethoxylated hydrogenated castor oil, polyoxyl 40 stearate, propylene glycol, and mixtures thereof.

4. The liquid oral dosage formulation of claim 1, wherein the pH of said formulation is between about 5.5 and about 6.2.

5. The liquid oral formulation of claim 4, wherein the pH of said formulation is about 5.9

6. The liquid oral formulation of claim 1 comprising a buffer system.

7. The liquid oral formulation of claim 6 wherein said buffer system comprises a member selected from the group consisting of alkaline metal citrate salts with citric acid, alkaline metal acetate salts with acetic acid, alkaline metal succinate salts with succinic acid, and mixtures thereof.

8. The liquid oral formulation of claim 7, further comprising an antifoaming agent.

9. The liquid oral formulation of claim 7, further comprising a preservative.

10. The liquid oral formulation of claim 9, wherein said preservative is a member selected from the group consisting of benzoic acid, sorbic acid, butylparaben, ethylparaben, methylparaben, propylparaben, sodium benzoate, sodium propionate, and mixtures or salts thereof.

11. A method for preparing a liquid oral solution comprising valsartan, comprising:
admixing water, wetting agent, preservatives, sweetener, flavouring agent and buffer components
adding valsartan with heat to yield a solution.

12. The method of claim 11, wherein said liquid oral solution has a pH of between about 5.5 and 7.0.

13. The method of claim 12, wherein said buffer system components are citric acid and sodium citrate.

14. A method for minimizing the degradation of an aqueous solution of valsartan, comprising providing an aqueous solution of valsartan and adjusting the pH of said solution to between about 4.5 and about 7.0.

15. A method for treating an angiotensin II mediated disorder or condition comprising administering an effective amount of a liquid oral dosage formulation comprising valsartan to a patient in need thereof, wherein the pH of said formulation is between about 4.5 and 7.0.
